# EUROPEAN PATENT SPECIFICATION

(11) **EP 1 968 512 B1**
(45) Date of publication and mention of the grant of the patent: **06.11.2013**
(21) Application number: 06841602.3
(22) Date of filing: 22.12.2006
(51) Int. Cl.: A61F 13/15

(54) **DISPOSAL AND PACKAGING DEVICE**
ENTSORGUNGS- UND VERPACKUNGSVORRICHTUNG
DISPOSITIF D'ÉLIMINATION ET D'EMBALLAGE

(30) Priority: 03.01.2006 GB 0600024; 09.06.2006 GB 0611398; 07.10.2006 GB 0619908
(43) Date of publication of application: 17.09.2008
(73) Proprietor: Silcott, Martha Selene, London Greater London N16 7AB (GB)
(72) Inventor: Silcott, Martha Selene, London Greater London N16 7AB (GB)
(74) Representative: Gallafent, Richard John
(86) International application number: PCT/EP2006/070180
(87) International publication number: WO 2007/077172

(56) References cited:
- EP-A- 1 262 134
- EP-A1- 1 413 272
- US-A- 4 838 327
- US-A- 6 059 100
- US-A1- 2002 029 546
- US-A1- 2005 098 466
- US-A1- 2005 228 354

## Description

The present invention relates to a disposal device suitable for the receipt of an item. It may be configured for use as packaging and/or storage of the item.

There are many situations in everyday life where manual disposal of soiled items is required which may be inconvenient, unhygienic or problematic in some other way, for example, by disposal in an environmentally unfriendly way. Such difficulties may particularly arise in relation to the disposal of sanitary products or of products contaminated with bodily fluid, for example, in the medical environment. Similarly, it may be difficult to dispose of products that have been exposed to chemical matter in, for example, the chemical or food industry. Alternatively, it may be difficult to receive forensic or medical items within a device without 'contaminating' the forensic or medical information carried thereby.

When manually disposing of a soiled item, it is possible that a person may be holding the item whilst attempting to manipulate a disposal device such as a bin and so have only one free hand available for assisting in the disposal process, for example, by opening the bin. Alternatively, it may be that the person does not want to touch the soiled item with more than one hand, to prevent contamination of other articles or if, for example, wearing only one glove. Further problems may arise if it is necessary to carry the soiled item, for example, in the absence of bins. One particular problem arises in the disposal of soiled tampon, tampon applicator or sanitary towel products where the woman will typically remove the soiled tampon, tampon applicator or sanitary towel from her vaginal region with one hand, thereby leaving only one free hand to assist further in the disposal process by for example, opening a receptacle (e.g. a bin or bag) for the receipt of the soiled item.

It is known to provide plastic bags for such purposes where two handles of the bag have to be tied together to close the bag to prevent the soiled tampon or sanitary towel from escaping. Such prior art plastic bags are, however, difficult to extract and open with one free hand to ensure effective receipt of the soiled item without in particular, soiling or contaminating the hands or clothing of the user during the bag opening and/or soiled item receiving process.

Receptacles for the receipt of soiled tampons are disclosed in US-A-4838327, US-A-6059100 and US-A-2005/0098466. None of the receptacles described in these specifications is easy to open with one hand. Receptacles for disposing of soiled diapers are disclosed in US-A-2002/0029546 and US-A-2005/0228354. Neither is easily opened with one hand.

The present invention seeks to provide a disposal device that is convenient and hygienic for use by the user and which can be manipulated with one hand for receipt of an item.

The present invention is directed to a disposal device for the receipt of a soiled item, the disposal device comprising a container substantially impervious to liquid having a mouth formed therein through which a soiled item may be received by the container, wherein in use, the mouth is arranged to be capable of manipulation by a single hand of a user between a mouth open position and a mouth closed position by mouth manipulation means located on opposite outside walls of the container for manipulating the mouth between a mouth open position and a mouth closed position, the mouth manipulation means comprising first and second loops through which one or more fingers or a thumb of the user can pass, and each loop being capable of adapting both a first configuration in which the loop profile is closed and a second configuration in which the loop profile is open, and characterised by one or more folds provided in the material of the loops such that each loop readily adopts the second configuration in the absence of external pressure on the loop.

The present invention provides a disposal device. The term 'disposal device' in used to mean a device that is suitable for the receipt of an item which is intended for disposal. The item may be a soiled item, for example, a soiled tampon, sanitary napkin, medical item or condom, which is received by the device for disposal thereof (e.g. in a waste bin or incinerator). Alternatively it may be, for example, a forensic or medical item that may be soiled in the sense of not being sterile, but rather is soiled (or contaminated) with material containing information of a forensic or medical quality (e.g. DNA information), and which is received by the device for stored receipt thereof e.g. for later inspection and analysis. Thus, in particular examples of these other embodiments the disposal device may function as a 'sample bag' for disposed receipt of forensic or medical items. In other embodiments, that item is unsoiled (e.g. in the sense of not having being used or in the case of for example, medical products, being sterile) and the device is primarily used for packaging and/or storage of the item. Thus in embodiments, a plethora of items such as food stuffs, nuts and bolts and haberdashery items, may also be stored/carried in the device and after use, potentially subsequently disposed of in the device.

A particular area where such a disposal device may be required is in the disposal of soiled sanitary or medical products. Thus, the present invention addresses a problem for a large proportion of the human population.

Typically, sanitary products, for example, tampons and sanitary towels are disposed of by either flushing them down the lavatory into the public sewerage system or by disposing of them in sanitary bags or bins which are then passed into the public refuse disposal system. Bins which process the soiled item mechanically before subsequent disposal are also known.

Disposal through the public sewerage system is highly undesirable from an environmental point of view due to the need to process sewage containing the sanitary products which may involve physical separation and in some cases, sanitary products passing beyond the sewage process into open water. Physical separation in the sewage process adds to cost and complexity. Moreover, disposal of sanitary products down the lavatory frequently causes blockages of lavatory systems, leading to inconvenience, expense and potential embarrassment.

Disposal using bins, bags or the like presents other problems. For example, when disposing of a soiled sanitary product, it is preferable for the soiled item to come into contact with only one hand of the person. Thus allowing the person to use the other hand to manipulate other objects like bin lids, bags or taps. Therefore it is preferable that the person should be able to manipulate the disposal device with one hand while holding the soiled item in the other. This enables the person to prevent contamination of other objects around them that may be touched prior to the person having the opportunity to clean their hands.

When used for the disposal of sanitary products, the disposal device avoids the risk of causing blockage to the lavatory system, avoids environmental problems associated with removal of the sanitary product from sewage systems and provides a simple, convenient, discreet and hygienic means of disposal of sanitary products. This may be especially advantageous when using facilities that are available to a number of people, for example, in hotels or in a work place. Cleaning of disposal bins may also be made more convenient and less distasteful as the disposal device of the present invention may be placed in a bin without risk of soiling the bin itself.

There is provided a disposal device for the receipt (e.g. disposed) of a soiled item. The soiled item may in particular be one that has come into contact with bodily fluids such as a soiled tampon, sanitary napkin, condom, medical item or forensic item. From a hygiene standpoint, effective receipt (e.g. for disposal or sample storage) of such soiled items is desirable. From a user standpoint, the device should enable effective receipt of the soiled item without risk of mess or contamination by (or, indeed, of) the soil carried by the item.

The disposal device comprises a container that is substantially impervious to liquid. The container is suitably in the form of a bag, sachet or pouch. Tapered and concertina bag, sachet of pouch forms are also envisaged. Gusseting may also be provided to the bag, sachet or pouch to enhance the available volume for disposal of the soiled item.

The container is suitably comprised of a plastic polymeric material (e.g. polythene) or a treated (e.g. waxed) paper material. The container is suitably formed from a biodegradable material, which however still functions to provide the necessary liquid imperviousness during soiled item disposal.

The container has a mouth formed therein through which a soiled item may be received by the container. In use, the mouth is arranged to be capable of manipulation by a single hand of a user between a mouth open position and a mouth closed position. The device further comprises mouth manipulation means located on an outside wall of the container for manipulating the mouth between the mouth open position and mouth closed position.

In use of the device, a person may, using their fingers or thumb, use the mouth manipulation means to pull apart one part of the mouth from the other to open the container. A soiled item that is being held in the other hand may then be deposited into the container through the mouth for receipt within the container.

The mouth manipulation means comprises a first and second finger-receipt means through, into or against which one or more fingers, or a thumb can be received. The first and second finger-receipt means are located on outside walls of the container and are so arranged to enable a person, in use, to insert at least one finger or thumb through, into or against each of the finger-receipt means to manipulate open the mouth of the container.

Suitably, the first and second finger-receipt means are arranged on either side of (e.g. adjacent to) the mouth.

Suitably, the first finger-receipt means is arranged to accommodate the thumb of a user and the second finger-receipt means is arranged to accommodate the index finger of a user. The mouth manipulation action (i.e. mouth opening/closing) is thus, achieved by a thumb and index finger spacing/de-spacing action.

The mouth manipulation means comprises first and second loops through which one or more fingers or a thumb can be inserted. The loops are located on the outside walls of the container and arranged to enable a person, in use, to insert at least one finger or thumb through the loop to manipulate open the mouth of the container. The first and second loops are arranged on either side of (e.g. adjacent to) the mouth.

The loops are sized and shaped for ready receipt of a user's thumb/finger (e.g. thumb in first loop and index finger in second loop). Suitably, the profile of the finger-receipt each loop is shaped to mirror the flattened/ovular end profile of a user's thumb or finger. That is to say, the loop profile (i.e. the aperture, which the loop defines) is slightly elongate for example, defining a generally ovular, parallelogram or rectangular loop profile.

In accordance with the invention, the or each loop is arranged such that it may readily adopt both a first 'storage' configuration, in which the loop profile is closed (i.e. the loop is flattened) and a second 'in use' configuration, in which the loop profile is open (i.e. to define the desired loop profile for finger/thumb receipt). The advantage of such arrangement is that the loops may readily be stored flat (e.g. within packaging or in a dispenser pack) but open up to provide the desired loop profile in use. This is achieved by providing one or more folds in each loop such that the loop readily adopts (e.g. 'pops out' into) the 'in use' configuration in the absence of any external pressure on the loop (e.g. as may exist when the device is stored in a dispenser pack or other packaging). Desirably, any dispenser device for the disposal device is adapted to allow at least one loop to 'pop out' from a lead disposal device when the dispenser device is in a disposal device dispensing position.

In one aspect, the loops are formed as an integral part of the container (e.g. a continuation of it). In another aspect, the loops seal to an outside wall of the container. The loops may either comprise the same material as that of the container or be formed from a different material. In one aspect, the mouth of the container is provided with lips that may act in use, to guide the receipt of the soiled item through the mouth. The lips may also to an extent act such as to shield the user's fingers/thumb (particularly those that are within the finger-receipt means) from contamination by the soiled item. Suitably, the mouth manipulation means (whose primary purpose is for manipulating the mouth between the mouth open position and mouth closed position) are further arranged for manipulating the lips. In particular, the mouth manipulation means enable manipulation of the lips to a lips open (and guiding) position when the mouth is in the mouth open position.

Embodiments are envisaged in which the mouth manipulation means and the lips form part of an integral arrangement. In one particular aspect, a first loop extends from a first lip and a second loop extends from a second lip. As will be understood from the later description, this kind of integral lips/loops arrangement is desirable from a manufacturing simplicity standpoint. Suitably, the lips may be manipulated into the lips open (and guiding position) before the mouth itself is manipulated open. Thus, in a typical use sequence the mouth manipulation means are employed to open the lips, and then to open the mouth. Where a reversible seal is provided to the mouth, the latter step may involve unsealing the mouth to open it up.

Preferably, the container is provided with a seal such that a substantially liquid tight seal to the container may be formed. Thus in a typical use process, once the soiled item has been placed in the container, the mouth is closed and the seal applied. In one aspect herein, two boundaries of the mouth can be brought together with the same fingers or thumb to form a seal.

The hygienic seal may be formed by any known methods, for example, using an adhesive strip on at least one internal boundary (e.g. edge) of the mouth. Alternatively, different materials that mesh together can be provided on each respective internal boundary of the mouth. For example, a hook and loop arrangement may be used where the material on the internal boundary consists of miniature plastic hooks for mouth manipulation with material located on the opposite inner boundary. Alternatively, a tongue and groove arrangement may be used, where a strip having a tongue is provided along one internal boundary of the mouth with a strip having a corresponding groove provided along the other internal boundary of the mouth.

In one aspect, the hygienic seal is arranged to be reversible (i.e. it may be sealed and then subsequently unsealed). In another aspect, the hygienic seal is arranged such that once sealed the seal cannot be broken without damaging the integrity of the container. The latter aspect may be employed in a tamper proof disposal device, which is designed for receipt of the soiled item and permanent sealing - any subsequent removal of the item would be by severing open (or otherwise damaging the integrity of) the container.

In a preferred embodiment, the mouth of the container is releasable and resealable as, preferably, the mouth is closed prior to use and opened and then re-closed during use and the adhesive desirably remains effective to form a seal on re-closing the mouth. This form of container may be desirable when used while a person is travelling, for example.

The container may be of any shape and size, however, it should be suitably proportioned to receive the items for which it is intended to be used. For sanitary products, it is desired that the container is of such size and shape so as to be able to receive a tampon and/or a sanitary towel and also not too large to be uncomfortable to carry on the person, for example in a hand bag or briefcase. As an example, the container may have a rectangular front side and a rectangular back side which are joined along three of the four boundaries. The fourth boundary generally defines the mouth so that the device is a generally rectangular sachet or pouch. Lip features may also be provided to the mouth of that sachet or pouch.

In a preferred aspect, the container adopts the form of a generally rectangular pouch, which is designed to lie flat in a 'storage' configuration. The generally rectangular pouch therefore has two opposing major (i.e. longer) sides and two opposing minor (i.e. shorter) sides. Suitably, the mouth is formed at one minor side of the generally rectangular pouch. Suitably, the generally rectangular pouch is provided with gusseting at the major sides of the rectangle, which define the length of the pouch. The gusseting may run parallel to the major sides or may be provided at a slight angle (e.g. 10 to 20°) thereto.

In embodiments, the disposal device is arranged for ease of folding into a folded-up state. Preferably, when the device is in the folded state one or more of the loops protrude therefrom such that the loops are readily able to receive a user's fingers (e.g. for use in unfolding of the disposal device from its folded-up state).

Preferably, the container will have a height of between 5 cm and 25 cm and a width of between 2 cm and 12 cm. Preferably, the container may itself be perfumed or contain a perfume as desired.

Preferably, the container is made of an impervious material that is resistant to the transmission of soil and odours from the soiled item. For example, the container may be made of a plastic or waxed material. When used for the disposal of sanitary products, preferably the container is impervious to aqueous liquid materials.

As a result of the minimal number of parts required to make this disposal device, the cost of production is low and allows the device to be produced in high volume.

In a preferred embodiment, the container used in the disposal of sanitary products is opaque. However, for other applications, for example, in the disposal of medical products or food products, the container may be translucent or transparent as desired. The container may be coloured or colourless. The container may have printed subject matter applied thereto (e.g. to one or more walls thereof).

In embodiments, the disposal device herein is also configured to act as a packaging device for the item in its pre-soiled state. Thus, in one usage scenario the item is supplied within the packaging and disposal device in a pre-usage state; then removed from the packaging and disposal device and used in some way, which usage results in soiling thereof; and then the soiled item is placed into the packaging and disposal device for disposal thereof.

In embodiments, the packaging and disposal device is provided with a second seal, which typically locates to seal off access to the mouth. Thus, to access an item within the packaging and disposal device the second seal must be broken to allow access to and opening of the mouth such that the item may be removed from the device through the opened mouth.

In embodiments, the second seal is configured to be non-resealable (i.e. once the second seal is broken it may not be resealed). In embodiments, the second seal is a tear-off (i.e. tear-away) seal, which typically has perforations to guide the tearing-off process. Suitably, the second seal is a tamper-evident seal. In other embodiments, the second seal is a re-sealable seal.

Disposal devices according to the present invention may be stored in a dispenser device which may include a housing which is arranged to accommodate a plurality of disposal devices, each of which is in a 'storage' configuration. Suitably, the dispenser device enables dispensing of one of said plural disposal devices at a time (i.e. serial dispensing). Suitably, the dispenser device is arranged such that on dispensing the mouth manipulation means, i.e. the loops are presented to the user for ready interaction with the user's fingers and/or thumb. Suitably, the dispenser device is shaped for ready accommodation in the user's hand (e.g. within the user's palm) and for such ready interaction of the user's fingers and/or thumb with a mouth manipulation means of the disposal device that is being dispensed therefrom.

In one aspect, the dispenser device is arranged for stacked storage (i.e. one of top of the other) of plural disposal devices herein. Suitably, in the stacking arrangement each disposal device lies flat, although the uppermost disposal device of the stack (i.e. that one which has no other disposal device lying thereon) may be arranged such as to present at least one loop away therefrom for ready interaction with the user's fingers and/or thumb. The dispenser device may further include one or more flaps or other suitable means for compressing the disposal devices in the stack to ensure maintenance of the flat stacking arrangement and/or to ensure that only one disposal device is dispensed at a time.

In another aspect, the dispenser device is arranged for rolled-up storage (i.e. in a rolled configuration) of plural disposal devices herein. Suitably, the plural disposal devices are arranged in a joined up series (i.e. the tail end of one joining to the lead end of another) with said join being broken as one disposal device is torn or ripped off from the roll on dispensing thereof. The lead disposal device of the roll (i.e. that one which is outermost in the roll) may be arranged such as to present at least one manipulation means away therefrom for ready interaction with the user's fingers and/or thumb. A 'snail shell' form dispenser is herein envisaged.

Embodiments of the present invention will hereinafter be described, by way of example, with reference to the accompanying drawings, in which:
Figure 1 is a perspective view of a first embodiment of a disposal device of the present invention in the lips and mouth closed position;
Figure 2 is a side view of the first embodiment of Figure 1 in the lips open, but mouth closed position;
Figure 3 is a perspective view of the top part of the first embodiment of Figure 1 shown in combination with a user's thumb/finger in the lips open, but mouth closed position of Figure 2;
Figure 4 is a view from above of the top part of the first embodiment of Figure 1 shown in combination with a user's thumb/finger in the lips open, but mouth closed position of Figure 2;
Figure 5 is a view from above of the top part of the first embodiment of Figure 1 shown in combination with a user's thumb/finger in a lips open and mouth part-open position;
Figure 6 is a view from above of the top part of the first embodiment of Figure 1 shown in combination with a user's thumb/finger in a lips open and mouth wide-open position;
Figure 7 is a perspective view of a second embodiment of a disposal device of the present invention in the lips and mouth closed position;
Figure 8 is a perspective view of a third embodiment of a disposal device of the present invention in the lips and mouth closed position;
Figure 9 is a perspective view of a fourth embodiment of a disposal device of the present invention in the mouth closed position;
Figure 10 is a side view of the fourth embodiment of Figure 9;
Figures 11A and 11B show perspective views of a fifth embodiment of a disposal device of the present invention in respectively mouth closed and mouth open positions;
Figure 12 shows a perspective view of an sixth embodiment of a disposal device of the present invention in the mouth closed position;
Figure 13 shows a perspective view of a first dispenser for dispensing a disposal device of the present invention;
Figures 14A to 14C show perspective views of a second dispenser for dispensing a disposal device of the present invention in respectively 'dispenser closed', 'dispenser part-open' and 'dispenser open' positions.

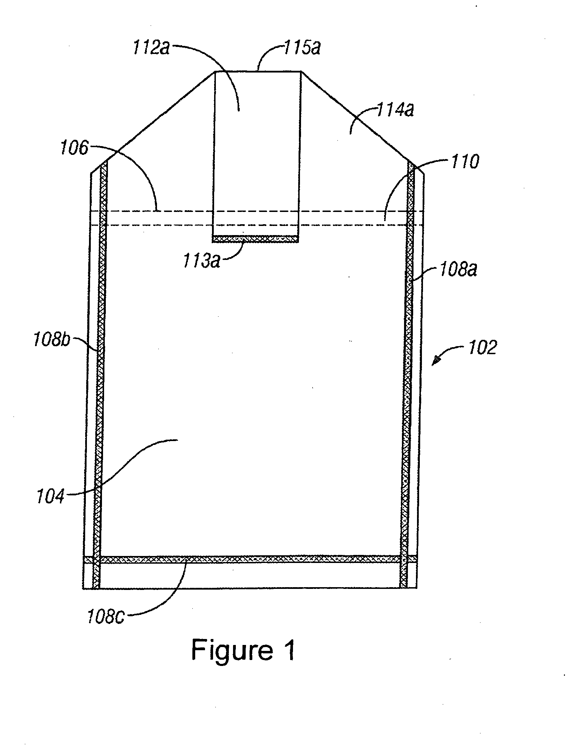

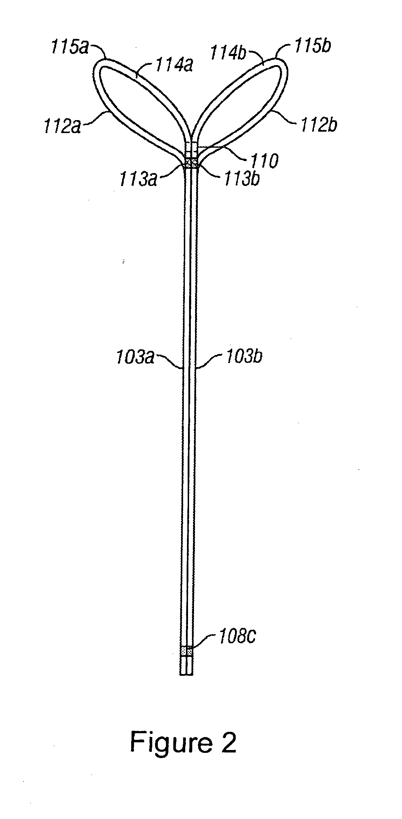

Figures 1 and 2 show an embodiment of a disposal device according to the present invention, denoted 102. It adopts the storage form of a flat pouch, and in an in-use configuration is suitable for the disposal of a soiled item (not shown). The disposal device defines a container 104, which is formed by the joining together of two flat plastic polymer sheets 103a, 103b by means of first, second and third permanent seal lines 108a, 108b and 108c and adhesive seal line 110. The adhesive seal line 110 defines a reversibly sealable (i.e. unseal/seal) barrier to the container 104 and the top (i.e. outer) edge thereof generally corresponds to the mouth 106 of the container 104.

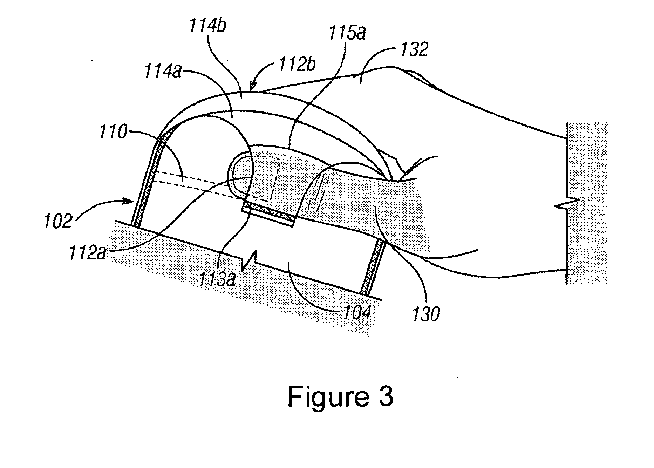

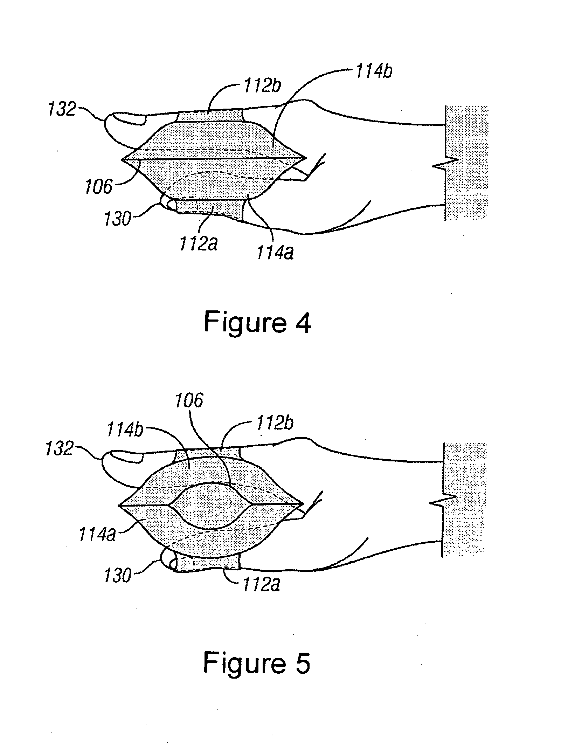

First and second lips 114a, 114b are provided to the mouth 106 and these are moveable to a 'lips open' configuration (see Figures 3 and 4) in which the lips form a guide for the receipt of a soiled item through the mouth 106 and into the container 104. First and second loops 112a, 112b are further provided to the mouth 106 and lips 114a, 114b. It will be noted that each loop 112a, 112b extends from an outer edge 115a, 115b of a respective lip 114a, 114b and attaches to an outside wall of the container 104 by means of respective permanent seal line 113a, 113b. It will further be noted that each permanent seal line 113a, 113b runs parallel to the adhesive seal line 110 and locates just underneath that adhesive seal line 110. It will be further noted that the loop profile (i.e. the shape of the loop aperture) defined by each loop 112a, 112b is generally elongate (i.e. elongate oval) to enable ready receipt of a user's thumb/fingers.
Figures 3 and 4 show the disposal device of Figures 1 and 2 at a first stage of a use operation. First loop 112a receives the user's thumb 130 and second loop 112b receives the user's index finger 132. The lips 114a, 114b are thereby, partly opened to give access to the mouth 106, which remains closed at this first stage.
Figure 5 shows a second stage in the use operation, in which the user's thumb 130 and index finger 132 have been separated somewhat to cause the seal 110 to be broken and to allow the mouth 106 to open slightly. The lips 114a, 114b are now wide open and provide a guiding passage for the insertion of a soiled item into the mouth 106.

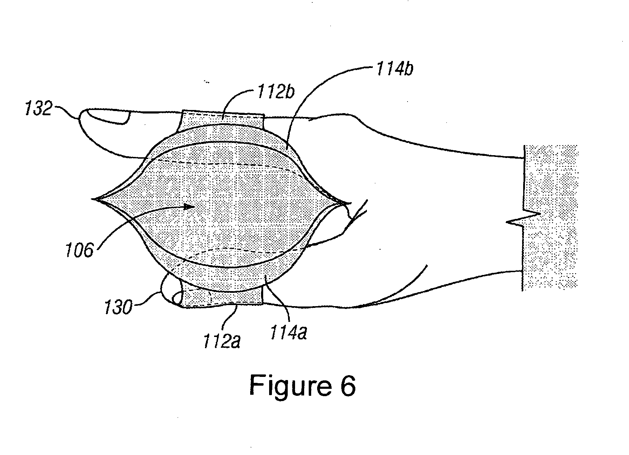

Figure 6 shows a third stage in the use operation, in which the user's thumb 130 and index finger 132 have been separated more widely to cause the mouth 106 to open broadly. The lips 114a, 114b are now even more wide open and continue to provide a guiding passage for the insertion of a soiled item into the now gaping mouth 106.

Once the soiled item has been placed into the container 104 through the mouth 106 thereof the user will bring his/her thumb 130 and index finger back together again. That is to say, the top of disposal device will return via a 'mouth slightly open' position as shown at Figure 5 to a 'mouth closed' position as shown at Figures 3 and 4. The user then employs his/her thumb 130 and index finger 132 to press against the adhesive seal line 110 to reseal the container 104. The soiled item is thereby, contained in sealed fashion within the container 104.

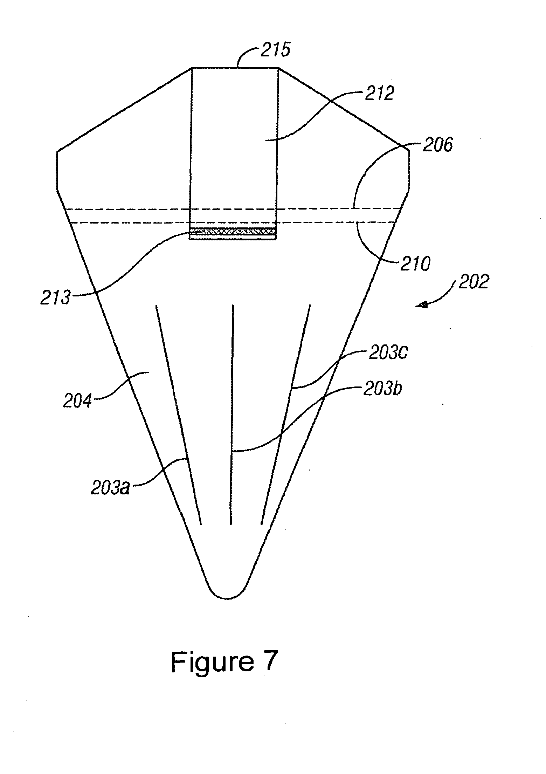

Figure 7 shows an alternative embodiment of a disposal device 202 herein, which may be appreciated to be a variation of the embodiment shown in Figures 1 to 6. The disposal device 202 defines a container 204, which is formed by a tapering bag defined in part by tapered fold lines 203a, 203b, 203c. As before, adhesive seal line 210 defines a reversibly sealable (i.e. unseal/seal) barrier to the container 204 and the top (i.e. outer) edge thereof generally corresponds to the mouth 206 of the container 204.

Lips 214 are again provided to the mouth 206 and as before, these are moveable to a 'lips open' configuration in which the lips form a guide for the receipt of a soiled item through the mouth 206 and into the container 204. Loops 212 are further provided to the mouth 206 and lips 214. As before, it will be noted that each loop 212 extends from an outer edge 215 of a respective lip 214 and attaches to an outside wall of the container 104 by means of respective permanent seal line 213. It will further be noted that each permanent seal line 213 runs parallel to the adhesive seal line 210 and locates just underneath that adhesive seal line 210.

The general operation of the fourth embodiment of Figure 7 closely mirrors that of the third embodiment of Figures 1 to 6 and is therefore, not described further.

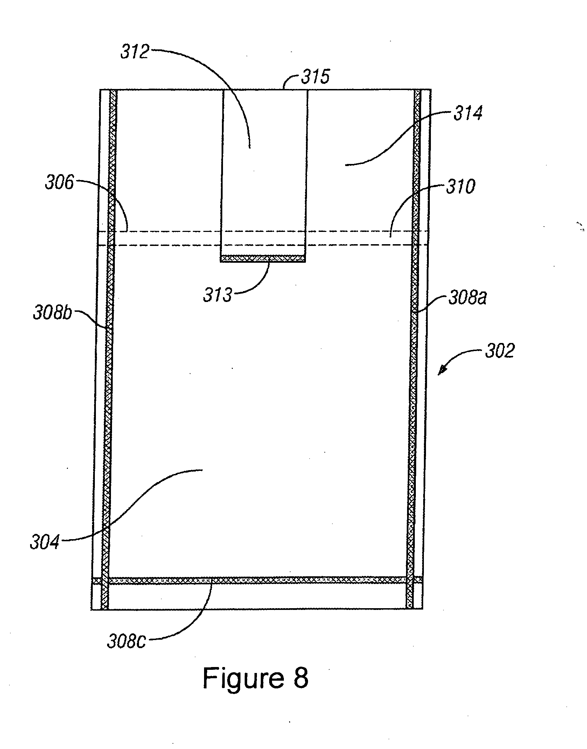

Figure 8 shows a further embodiment of a disposal device 302 herein, which may be appreciated to be another variation of the third embodiment of Figures 1 to 6. The disposal device 302 adopts the storage form of a flat pouch and in an in-use configuration is suitable for the disposal of a soiled item (not shown). The disposal device defines a container 304, which is formed by the joining together of two flat plastic polymer sheets by means of first, second and third permanent seal lines 308a, 308b and 308c and adhesive seal line 310. The adhesive seal line 310 defines a reversibly sealable (i.e. unseal/seal) barrier to the container 304 and the top (i.e. outer) edge thereof generally corresponds to the mouth 306 of the container 304.

Lips 314 are provided to the mouth 306 and these are moveable to a 'lips open' configuration in which the lips form a guide for the receipt of a soiled item through the mouth 306 and into the container 304. Loops 312 are further provided to the mouth 306 and lips 314. It will be noted that each loop 312 extends from an outer edge 315 of a respective lip 314 and attaches to an outside wall of the container 304 by means of respective permanent seal line 313. It will further be noted that each permanent seal line 313 runs parallel to the adhesive seal line 310 and locates just underneath that adhesive seal line 310. The loop profile (i.e. the shape of the loop aperture) defined by each loop 312 is generally elongate (i.e. elongate oval) to enable ready receipt of a user's thumb/fingers.

The general operation of the fifth embodiment of Figure 8 again closely mirrors that of the third embodiment of Figures 1 to 6 and is therefore, not described further.

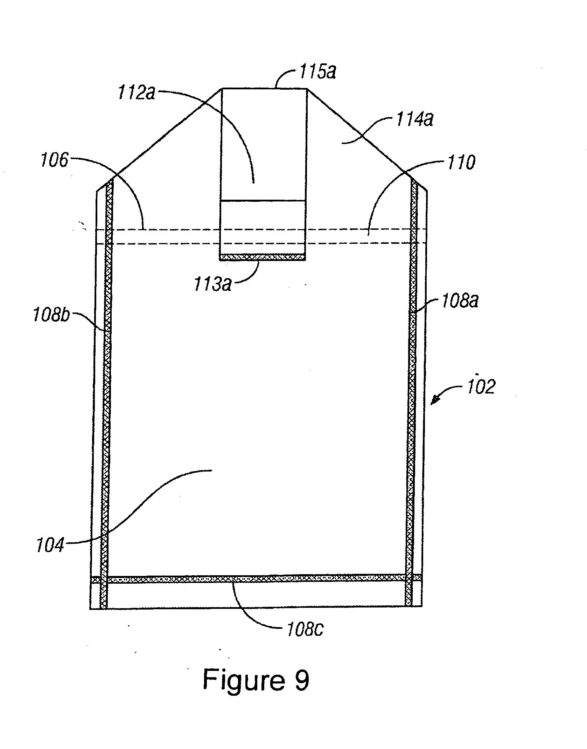

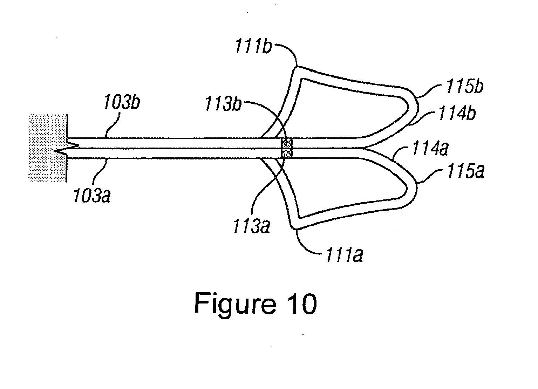

Figure 9 shows the fold 111a, 111b which is provided to each loop 112a, 112b. As may be seen by reference to Figure 10, which shows a top detail of the disposal device, the consequence of having this additional fold 111a, 111 b is that each loop 112a, 112b tends to 'pop up' into a lips 114a, 114b semi-open position in the absence of any pressure on the loop 112a, 112b. Thus, in effect the additional fold 111a, 111b tends to bias each loop 112a, 122b into such a semi-open position.

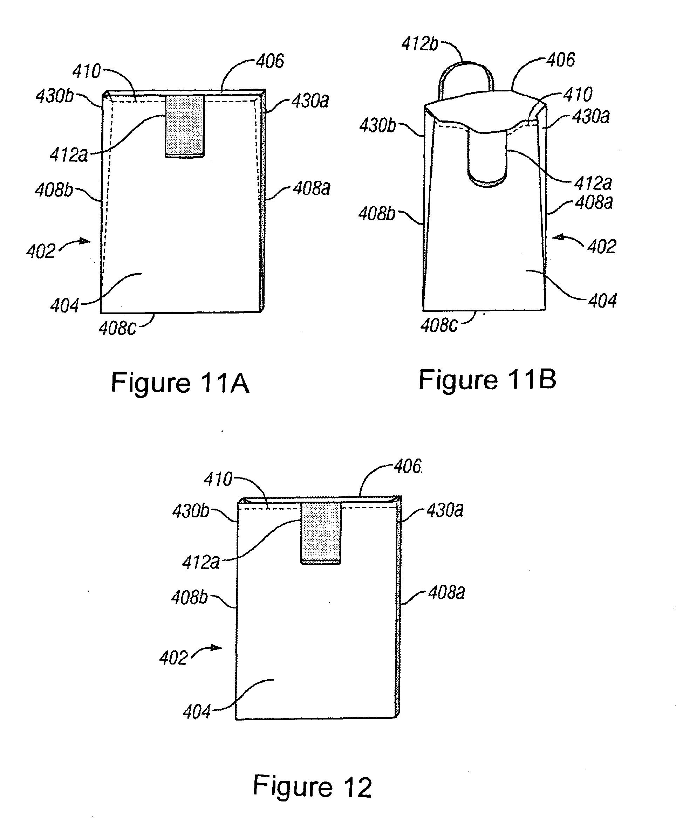

Figures 11A and 18B show a sixth embodiment of a disposal device 402 herein, which defines a rectangular pouch- form container 404. As seen at Figure 11A, the disposal device 402 adopts the storage form of a flat pouch. As seen at Figure 11B, an in-use configuration is suitable for the disposal of a soiled item (not shown). The rectangular pouch-form container 404 has major edges 408a, 408b which define its length; a lower minor edge 408c, which defines its base; and adhesive seal line 410. The adhesive seal line 410 defines a reversibly sealable (i.e. unseal/seal) barrier to the container 404 and the top (i.e. outer) edge thereof generally corresponds to the mouth 406 of the container 404. Loops 412a, 412b are provided to the mouth 406. The loop profile (i.e. the shape of the loop aperture) defined by each loop 412 is generally elongate (i.e. elongate oval) to enable ready receipt of a user's thumb/fingers.

Gussets 430a, 430b are provided at each major edge 408a, 408b of the container 404. The use of such gussets 430a, 430b is advantageous in that the effective volume of the container 404 for soiled item disposal is increased. It will be noted that each gusset 430a, 430b is inclined at angle of about 10° to its respective edge 408a, 408b such that the gusset 430a, 430b tapers towards the bottom of the pouch 404. It will be noted that the top part of each gusset 430a, 430b lies just below the adhesive seal line 410.
Figure 12 shows a seventh embodiment herein, which is identical to the sixth embodiment of Figures 11A and 11B other than that each gusset 430a, 430b runs parallel to its major edge 408a, 408b (i.e. it is not tapered).

The general principle of operation of the sixth and seventh embodiments of respectively Figures 11A and 11B and Figure 12 closely mirrors that of the previously described embodiments and is therefore, not described further.

Any of the embodiments described above is suitable for the disposed receipt of an item such as a condom, tampon, sanitary towel, medical item (not shown) for disposal thereof, or for the disposed receipt of a medical or forensic item (not shown) for later analysis thereof.

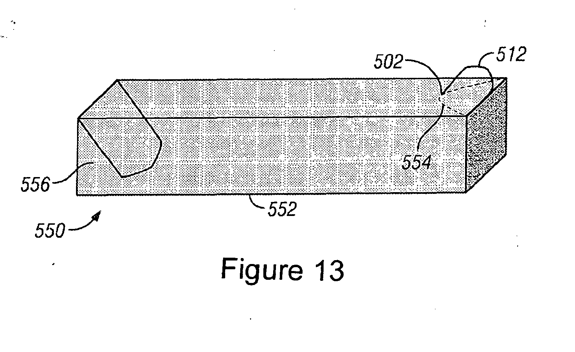

Figure 13 shows a first dispenser device 550 herein that is suitable for storage of plural disposal devices in a stacking arrangement and which enables serial (i.e. one at a time) dispensing of disposal devices from the stack.

The first dispenser device 550 comprises a rectangular box housing 552 (e.g. formed of cardboard). The housing 552 is provided with an opening 554 (which may in aspects, initially be provided with a tear-off (e.g. perforated seal) cover) through which a loop 512 of the lead disposal device 502 of the stack projects. In use, the user would hold the box housing 552 in a cupped hand in such a way as to allow for ready insertion of a finger or thumb into the projecting loop 512 of the lead disposal device 502. Once such finger/thumb has been inserted into the loop 512 the user would then pull the loop 512 upwards to thereby dispense the disposal device 502 from the box housing 552. The box housing 552 is further provided with an internal flap 556, which is arranged to apply downward pressure to the stack and thereby to both keep the disposal devices 502 in the stack in a flat configuration and to ensure that only one disposal device 502 is dispensed at a time.

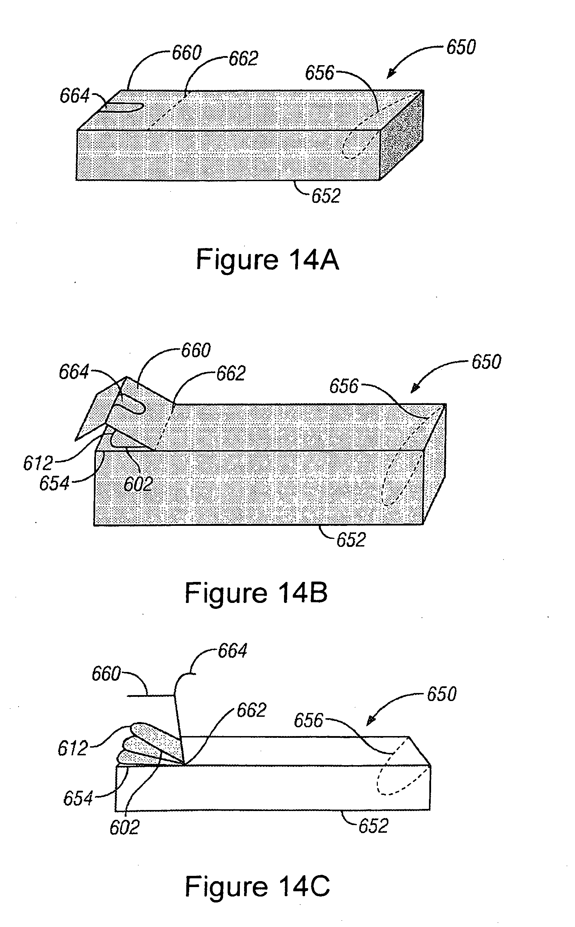

Figures 14A to 14C shows a second dispenser device 650 herein in successive states of operation. The second dispenser device 650 is suitable for storage plural disposal devices 602 in a stacking arrangement and which enables serial (i.e. one at a time) dispensing of disposal devices from the stack.

The second dispenser device 650 comprises a rectangular box housing 652 (e.g. formed of cardboard). The housing 652 is provided with an opening 654 that is provided with a lid 660. The lid 660 joins to the housing at fold 662 about which it hinges upwards into its lid open state (as shown at Figures 14B and 14C) tear-off. The lid 660 is further provided with an upwardly projecting tab 664, which assists in pulling open of the lid 660. Optionally and not shown, the housing 652 may be provided with an extra piece of cardboard into which the lid 660 folds in a 'lid closed' position. When the lid 660 is in its 'lid open' position loop 612 of the lead disposal device 602 of the stack projects from the opening 654. In use, the user would hold the box housing 652 in a cupped hand in such a way as to allow for pulling up of the tab 664 to open the lid 660. A finger or thumb would then be inserted into the projecting loop 612 of the lead disposal device 602. Once such finger/thumb has been inserted into the loop 612 the user would then pull the loop 612 up and away from the box housing 652 to thereby dispense the lead disposal device 602. The box housing 652 is further provided with an internal flap 656, which is arranged to apply downward pressure to the stack and thereby to both keep the disposal devices 602 in the stack in a flat configuration and to ensure that only one disposal device 602 is dispensed at a time.

## Claims

1. A disposal device for the receipt of a soiled item, the disposal device comprising a container substantially impervious to liquid having a mouth (106) formed therein through which a soiled item may be received by the container wherein, in use, the mouth (106) is arranged to be capable of manipulation by a single hand of a user between a mouth open position and a mouth closed position, by mouth manipulation means located on opposite outside walls of the container for manipulating the mouth (106) between a mouth open position and a mouth closed position, the mouth manipulation means comprising first and second loops (112a, 112b) through which one or more fingers or a thumb of the user can pass, and each loop (112a, 112b) being capable of adapting both a first configuration in which the loop profile is closed and a second configuration in which the loop profile is open, and **characterised by** one or more folds (111 a, 111 b) provided in the material of the loops such that each loop readily adopts the second configuration in the absence of external pressure on the loop.

2. A disposal device according to Claim 1 wherein the mouth (106) of the container is provided with lips (114a, 114b) for guiding the receipt of the soiled item into the mouth (106).

3. A disposal device according to Claim 1 or 2 wherein the mouth manipulation means and the lips form an integral arrangement.

4. A disposal device according to Claim 1 to 3 wherein the container is provided with a seal (310) such that a substantially liquid tight seal to the container may be formed.

5. A disposal device according to Claim 4 wherein a layer of adhesive is provided on at least one internal boundary of the mouth, such that when the mouth is in the mouth closed position, the mouth forms the seal.

6. A disposal device according to any one of Claims 1 to 5 further configured to act as a packaging device for the item in a pre-soiled state wherein the item is stored in the container prior to use, the disposal device further comprising a second seal that seals off access to the mouth (106).

7. A disposal device according to Claim 6 wherein the second seal is a tear-off seal.

## Patentansprüche

1. Eine Entsorgungsvorrichtung zur Aufnahme eines verschmutzten Stückes, die Entsorgungsvorrichtung bestehend aus einem weitgehend für Flüssigkeiten undurchlässigen Behälter mit einer darin geformten Öffnung (106) durch die ein verschmutztes Stück in den Behälter aufgenommen werden kann, wobei im Gebrauch die Öffnung (106) so angelegt wird, daß sie mit einer einzigen Hand durch beidseitig an den Außenwänden des Behälters gegenübergestellte angebrachte Öffnungsverstellungsvorrichtungen zur Manipulation der Öffnung (106) zwischen einer Position mit geöffneter Öffnung und einer mit geschlossener Öffnung zu manipulieren ist, die Öffnungsverstellungsvorrichtung bestehend aus einer ersten und einer zweiten Schleife (112a, 112b) durch die ein oder mehrere Finger oder der Daumen des Verwenders hindurch gehen kann, und jede Schleife (112a, 112b) dazu fähig ist, sowohl zur ersten Gestaltung angepaßt zu werden, in der der Schleifenprofil geschlossen und durch eine oder mehrere Falten (111a, 111b), im Stoff der Schleifen gekennzeichnet ist, so angelegt, daß jede Schleife ohne weiteres die zweite Gestaltung annimmt solange kein Außendruck auf die Schleife ausgeübt wird, als auch zur zweiten Gestaltung in der der Schleifenprofil offen ist.

2. Eine gemäß Anspruch 1 Entsorgungsvorrichtung in der die Öffnung (106) mit Lippen (114a, 114b) versehen ist, um bei Aufnahme eines verschmutzen Stückes dem Stück den Weg in die Öffnung (106) zu leiten.

3. Eine gemäß Ansprüche 1 oder 2 Entsorgungsvorrichtung in der die Manipulationsvorrichtung der Öffnung und die Lippen eine integriere Gruppierung bilden.

4. Eine gemäß Ansprüche 1 bis 3 Entsorgungsvorrichtung, in der der Behälter mit einem Verschluß vorgesehen wird, damit eine weitgehend für Flüssigkeiten dichter Verschluß zum Behälter gestaltet werden kann.

5. Eine gemäß Anspruch 4 Entsorgungsvorrichtung, in der eine Klebschicht an mindestens einer internen Rand der Öffnung vorgesehen wird damit die Öffnung in der geschlossenen Stellung den Verschluß bildet.

6. Eine gemäß irgend einer der Ansprüche 1 bis 5 Entsorgungsvorrichtung weiterhin so gestaltet, daß sie als Behälter für das unverschmutzte Stück vor dem Gebrauch verwendet werden kann, indern die Entsorgungsvorrichtung weiterhin mit einem zweiten Verschluß vorgesehen ist, der den Zugang zur Öffnung (106) abdichtet.

7. Eine gemäß Anspruch 6 Entsorgungsvorrichtung, in der der zweite Verschluß ein Abreißverschluß ist.

## Revendications

1. Dispositif d'élimination pour la réception d'un article souillé, le dispositif d'élimination comprenant un récipient sensiblement imperméable aux liquides comportant une ouverture (106) formée dans celui-ci à travers laquelle un article souillé peut être reçu par le récipient dans lequel, en utilisation, l'ouverture (106) est agencée pour pouvoir être manipulée par une seule main d'un utilisateur entre une position ouverte de l'ouverture et une position fermée de l'ouverture, par des moyens de manipulation d'ouverture situés sur des parois extérieures opposées du récipient pour manipuler l'ouverture (106) entre une position ouverte de l'ouverture et une position fermée de l'ouverture, les moyens de manipulation d'ouverture comprenant des première et deuxième boucles (112a, 112b) à travers lesquelles un ou plusieurs doigts ou pouces de l'utilisateur peuvent passer, et chaque boucle (112a, 112b) étant capable d'adopter à la fois une première configuration dans laquelle le profil de boucle est fermé et une deuxième configuration dans laquelle le profil de boucle est ouvert, et **caractérisé par** un ou plusieurs plis (111a, 111b) prévus dans le matériau des boucles de sorte que chaque boucle adopte facilement la deuxième configuration en l'absence d'une pression externe sur la boucle.

2. Dispositif d'élimination selon la revendication 1, dans lequel l'ouverture (106) du récipient est pourvue de lèvres (114a, 114b) pour guider la réception de l'article souillé dans l'ouverture (106).

3. Dispositif d'élimination selon la revendication 1 ou 2, dans lequel les moyens de manipulation d'ouverture et les lèvres forment un agencement intégral.

4. Dispositif d'élimination selon les revendications 1 à 3, dans lequel le récipient est pourvu d'un joint (310) de sorte qu'un joint sensiblement étanche aux liquides pour le récipient peut être formé.

5. Dispositif d'élimination selon la revendication 4, dans lequel une couche d'adhésif est prévue sur au moins une frontière interne de l'ouverture, de sorte que, lorsque l'ouverture est dans la position fermée de l'ouverture, l'ouverture forme le joint.

6. Dispositif d'élimination selon l'une quelconque des revendications 1 à 5, configuré en outre pour agir en tant que dispositif d'emballage pour l'article dans un état pré-souillé dans lequel l'article est rangé dans le récipient avant une utilisation, le dispositif d'élimination comprenant en outre un deuxième joint qui scelle un accès à l'ouverture (106).

7. Dispositif d'élimination selon la revendication 6, dans lequel le deuxième joint est un joint destiné à être enlevé par arrachement.
